(19) Europäisches Patentamt
European Patent Office
Office européen des brevets

(11) Veröffentlichungsnummer: **0 027 990**
**A1**

(12) # EUROPÄISCHE PATENTANMELDUNG

(21) Anmeldenummer: **80106395.9**

(22) Anmeldetag: **21.10.80**

(51) Int. Cl.³: **C 07 D 213/79**, C 07 D 213/80, A 61 K 31/455, A 61 K 31/44 // C07D213/85, C07D413/12

(30) Priorität: **26.10.79 DE 2943774**

(43) Veröffentlichungstag der Anmeldung: **06.05.81 Patentblatt 81/18**

(84) Benannte Vertragsstaaten: **AT BE CH DE FR GB IT LI LU NL SE**

(71) Anmelder: **SCHERING AKTIENGESELLSCHAFT Berlin und Bergkamen, Müllerstrasse 170/178 Postfach 65 0311, D-1000 Berlin 65 (DE)**

(72) Erfinder: **Strehlke, Peter, Dr., Droysenstrasse 10A, D-1000 Berlin 12 (DE)**
Erfinder: **Mannesmann, Gerda, Dr., Hagenplatz 3e, D-1000 Berlin 33 (DE)**

(54) **Substituierte Pyridine, Verfahren zu ihrer Herstellung und ihre Verwendung.**

(57) Es werden neue substituierte Pyridine der allgemeinen Formel I

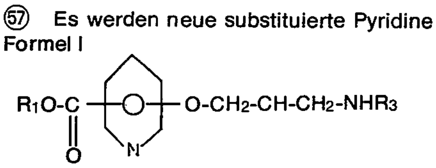

(I)

worin $R_1$ Wasserstoff, Alkyl mit bis zu 6 C-Atomen und Aralkyl mit 7-9 C-Atomen, $R_2$ Wasserstoff und Alkanoyl mit 2 bis 7 C-Atomen und $R_3$ Alkyl mit bis zu 6 C-Atomen bedeuten, sowie deren Additionssalze beschrieben, die eine starke und schnell einsetzende antihypertensive Wirkung zeigen.

EP 0 027 990 A1

Gegenstand der Erfindung sind substituierte Pyridine der Formel I, Verfahren zu ihrer Herstellung und ihre Verwendung als Arzneimittel mit blutdrucksenkender Wirkung gemäß den Ansprüchen.

Aus der DE-OS 2.556.110 sowie den US-PS 4.053.605, 4.091.104 und 4.092.419 sind bereits Cyanopyridine mit einem 3-Amino-2-hydroxypropoxyrest bekannt, die blutdrucksenkend wirken und außerdem ß-adrenerge Receptoren blockieren.

Es wurde nun gefunden, daß die substituierten Pyridine der Formel I die bekannten Cyanopyridine in ihrer blutdrucksenkenden Wirkung übertreffen. Insbesondere zeigen sie bereits bei geringeren Dosen an Wirksubstanz einen statistisch signifikaten Blutdruckabfall im pharmakologischen Test mit Versuchstieren.

Die Formel I umfaßt alle 10 positionsisomeren Verbindungen, die sich durch die verschiedene Stellung von zwei Substituenten am Pyridinring ergeben, wobei 2.5-disubstituierte Verbindungen bevorzugt sind. Unter Alkyl ist insbesondere der Methyl-, Äthyl-, n-Propyl-, Isopropyl-, n-Butyl-, Isobutyl-, tert.-Butyl, Pentyl- und der Hexylrest und unter Aralkyl der Benzyl-, 1- und 2-Phenyläthyl- und der 1-, 2- und 3-Phenylpropylrest zu verstehen.

Als Alkanoyl kommen an sich alle Reste von organischen Säuren infrage, soweit sie physiologisch verträglich sind. Insbesondere geeignet ist der Acetyl- und der Pivaloylrest.

Vorzugsweise ist $R_3$ eine Isopropyl- oder eine tert. Butylgruppe.

Die Verbindungen der Formel I können mit einem oder zwei Äquivalenten einer physiologisch verträglichen Säure Additionssalze bilden. Als Säuren können hier beispielsweise die anorganischen Säuren wie Halogenwasserstoffsäuren, insbesondere Salzsäure sowie Schwefelsäure, Phosphorsäure oder Salpetersäure verwendet werden. Als Beispiele für organische Säuren werden die Essigsäure, Propionsäure, Maleinsäure, Weinsäure, Benzoesäure oder Cyclohexylsulfaminsäure genannt.

Falls $R_1$ ein Wasserstoffatom ist, können auch Salze an der dann vorliegenden Carboxylgruppe gebildet werden. Beispielsweise seien hier genannt das Kalium-, Natrium- und das Ammoniumsalz oder die Salze organischer Basen wie das Glukamin, N-Methylglukamin, Äthanolamin, Diäthanolamin, Morpholin, Tris-(hydroxymethyl)methylamin usw.

Die Verbindungen der Formel I enthalten mindestens ein asymmetrisches Kohlenstoffatom und können damit sowohl in der racemischen (R,S)-Form als auch in der optisch akitven R- oder S-Form vorliegen. Die optisch aktiven Formen können durch die üblichen Racemat-Trennmethoden aus dem Racemat hergestellt oder auch durch chirale Ausgangsmaterialien gezielt synthetisiert werden. Die biologische Wirksamkeit einer

optisch aktiven Form, vorzugsweise der S-Form, die dem natürlich vorkommenden Adrenalin entspricht, kann dann größer sein als die des Racemats.

Die Herstellung der Verbindungen der Formel I erfolgt nach an sich bekannten Methoden und kann auf verschiedenen Wegen erreicht werden.

Bei der Methode a) wird der Hydroxyester der Formel II mit der äquivalenten Menge oder einem Überschuß des Epoxids der Formel III, vorzugsweise mit Epichlorhydrin, ohne oder mit einem inerten Lösungsmittel wie Dimethylformamid Dioxan oder Tetrahydrofuran in Gegenwart einer organischen Base wie Piperidin oder Triäthylamin oder einer anorganischen Base wie einem Alkalicarbonat bei Raumtemperatur bis Siedetemperatur der Lösung, vorzugsweise bei 20 - 100 °C, umgesetzt. Nach Isolation des Zwischenprodukts wird dieses mit dem entsprechenden primären Amin in einem geeigneten Lösungsmittel wie Methanol, Äthanol, Isopropanol oder Dioxan bei Temperaturen zwischen Raumtemperatur und Siedetemperatur, vorzugsweise bis 40 - 70 °C, zur Reaktion gebracht.

Bei der Umsetzung gemäß Methode b) werden äquivalente Mengen des Hydroxyesters der Formel II und des Azetidinols der Formel IV in Benzylalkohol bei 140 °C in Gegenwart einer katalyrischen Menge Kaliumhydroxid umgesetzt, wobei nach dem Verfahren der DE-OS 2 503 222 vorgegangen wird.

Die Umsetzung gemäß Methode c) erfolgt mit dem Alkalisalz des Hydroxyesters der Formel II, das aus dem Hydroxyester der Formel II und einem Alkalihydrid erhalten wird,

und dem aktivierten Oxazolidin der Formel V, vorzugsweise mit dem Tosylat, in einem polaren aprotischen Lösungsmittel wie Dimethylformamid, Dimethylacetamid oder Hexamethylphosphorsäuretriamid bei einer Temperatur zwischen Raumtemperatur und der Siedetemperatur des Reaktionsgemisches, vorzugsweise zwischen 100 und 150 °C. Das Zwischenprodukt wird mit einer verdünnten wässrigen Mineralsäure bei Temperaturen zwischen Raumtemperatur und Siedetemperatur, vorzugsweise bei 80 - 100 °C, behandelt.

Bei der Reaktion gemäß Methode d) wird eine Lösung äquivalenter Mengen des Halogenesters der Formel VI und von Glycidol in einem polaren aprotischen Lösungsmittel, wie bei Methode c) beschrieben, zur Suspension eines Alkalihydrids in dem selben Lösungsmittel bei Temperaturen zwischen -10 °C und 60 °C, vorzugsweise zwischen 0 °C bis 10 °C, gegeben und das Zwischenprodukt wie bei Methode a) mit einem primären Alkylamin umgesetzt. (vgl. J.Med.Chem. 21(1978)123).

Bei der Herstellung nach Methode e) wird ein Oxazolidin der Formel VIII mit einem Alkalihydrid in einem polaren aprotischen Lösungsmittel, wie bei Methode.c) beschrieben, bei Temperaturen zwischen Raumtemperatur und 100 °C zum Alkalisalz der Formel VIII umgesetzt und dieses bei Temperaturen zwischen Raumtemperatur und Siedetemperatur, vorzugsweise bei 20 °C bis 100 °C mit der äquivalenten Menge des Halogenesters der Formel VI zur Reaktion gebracht. Das Zwischenprodukt wird weiter wie bei Methode c) behandelt.

Die Überführung einer Verbindung der Formel I, in der $R_1$ Alkyl oder Aralkyl bedeutet, in eine Verbindung der Formal I, in der $R_1$ Wasserstoff bedeutet, erfolgt durch saure Hydrolyse mit einer Mineralsäure, vorzugsweise konzentrierter Salzsäure, bei erhöhter Temperatur, vorzugsweise bei 100 °C, oder durch alkalische Hydrolyse mit einer wässrigen oder alkoholischen Alkali- oder Erdalkalihydroxid-Lösung, wobei Bariumhydroxid bevorzugt wird, bei Temperaturen zwischen Raumtemperatur und Siedetemperatur, vorzugsweise bei Siedetemperatur.

Die Überführung einer Verbindung der Formel I, wobei $R_2$ Wasserstoff bedeutet, in eine Verbindung der Formel I, wobei $R_2$ Alkanoyl mit 2-7 C-Atomen bedeutet, erfolgt mit äquivalenten Mengen des entsprechenden Alkanoylhalogenids oder -anhydrids, vorzugsweise dem Säurechlorid, in Gegenwart eines Acylierungskatalysators, wie Pyridin oder 4-Dimethylaminopyridin, bei Temperaturen zwischen -10 °C und 100 °C, vorzugsweise bei 0 °C bis 20 °C.

Die Verbindungen der allgemeinen Formel I besitzen eine starke und schnell einsetzende antihypertensive Wirkung, die durch ihren gefäßerweiternden Effekt hervorgerufen wird. Außerdem blockieren die neuen Verbindungen die ß-andrenergen Receptoren und verhindern so die infolge des Blutdruckabfalls auftretende reflektorische Tachycardie.

Die erfindungsgemäßen Verbindungen werden nach an sich bekannten Methoden der Galenik zu Arzneimitteln, insbesondere für die orale Applikation, verarbeitet.

Die nachfolgenden Beispiele sollen die Erfindung erläutern.

Beispiel 1

a) zu 13,6 g Hydroxylamin-O-sulfonsäure in 100 ml Wasser werden 21,3 g 5-Benzyloxy-2-pyridincarbaldehyd (J.Med.Chem. 20(1977)1261) in 100 ml Methanol bei 0 °C getropft. Dann gibt man bei 0 °C 50 ml 2N Natriumcarbonat-Lösung und darauf 150 ml 2N Natronlauge dazu, rührt die Lösung mit 0,5 l Methylenchlorid aus, schüttelt nach Abtrennen der organischen Phase die wässrige Phase noch 2mal mit Methylenchlorid aus, wäscht die vereinigten organischen Phasen mit Wasser, trocknet mit Natriumsulfat und dampft ein. Nach Umkristallisation aus Äthanol erhält man 17,5 g 5-Benzyloxy-2-cyanpyridin; Schmelzpunkt 93-94 °C.

b) 17 g 5-Benzyloxy-2-cyanpyridin werden in 400 ml Methanol gelöst, mit 4,4 g Natriummethylat versetzt und 18 Stunden bei Raumtemperatur gerührt. Dann werden 200 ml 1N Salzsäure zugesetzt und die Lösung 10 Minuten auf dem Wasserbad erwärmt. Darauf wird das Lösungsmittel eingedampft, der Rückstand in Wasser gelöst, mit Kaliumcarbonat alkalisch gemacht und mit Äther 3mal extrahiert. Nach Trocknen, Eindampfen des Lösungsmittels und Umkristallisieren des Rückstands aus Cyclohexan erhält man 15,5 g 5-Benzyloxy-2-pyridincarbonsäuremethylester; Schmelzpunkt 85-87 °C.

c) 15 g 5-Benzyloxy-2-pyridincarbonsäuremethylester werden in 500 ml Äthanol mit 3 g Palladium/Kohle

0027990

(5 %) bei Normaldruck hydriert. Nach Aufnahme der berechneten Menge Wasserstoff wird der Katalysator abfiltriert, das Filtrat eingedampft und der Rückstand aus Isopropanol umkristallisiert. Man erhält 7,8 g 5-Hydroxy-2-pyridincarbonsäuremethylester; Schmelzpunkt 188-194 °C.

d) 4,45 g 5-Hydroxy-2-pyridincarbonsäuremethylester werden in 50 ml Dimethylformamid gelöst und mit 870 mg Natriumhydrid/Ölsuspension (80 %ig) versetzt. Nach Beendigung der Wasserstoffentwicklung werden 11,3 g 3-tert.Butyl-5-(4-methylphenylsulfonyloxymethyl)-2-phenyloxazolidin in 50 ml Dimethylformamid zugegeben und das Gemisch 12 Stunden im Rückfluß gekocht. Dann wird das Lösungsmittel im Vakuum eingedampft und der Rückstand mit 107 ml 1N Salzsäure 85 Minuten bei 60 °C gerührt. Nach Abkühlen wird 2mal mit Äther extrahiert, die wässrige Phase im Vakuum eingedampft und der Rückstand nach Behandeln mit Kohle in Äthanol aus Äthanol/Äther umkristallisiert. Man erhält 2,5 g 5-(3-tert. Butylamino-2-hydroxypropoxy)-2-pyridincarbonsäuremethylester, Dihydrochlorid; Schmelzpunkt 152-163 °C.

## Beispiel 2

20 g 5-Hydroxy-2-pyridincarbonsäuremethylester (Beispiel 1c) werden mit 16,8 g 1-tert. Butyl-3-azetidinol und 730 mg Kaliumhydroxid in 200 ml Benzylalkohol 5 Stunden unter einer Stickstoffatmosphäre auf 140 °C erwärmt. Nach Erkalten wird mit Äther versetzt und die Lösung 2mal mit 1N Salzsäure extrahiert. Die

Wasserphase wird mit Kaliumcarbonat alkalisch gemacht und 2mal mit Äther und 2mal mit Methylenchlorid extrahiert. Diese organischen Phasen werden vereinigt, über Natriumsulfat getrocknet und eingeengt. Der Rückstand wird mit methanolischer Salzsäure in das Hydrochlorid überführt, das aus Äthanol umkristallisiert wird. Man erhält 13,7 g 5-(3-tert. Butylamino-2-hydroxypropoxy)-2-pyridincarbonsäurebenzylester, Hydrochlorid vom Schmelzpunkt 175-179 °C.

Beispiel 3

15,5 g des Benzylesters aus Beispiel 2 werden mit 150 ml konz. Salzsäure 3 Stunden bei 100 °C behandelt. Dann wird auf Eiswasser gegossen, mit Äther 3mal extrahiert und die wässrige Phase eingedampft. Der Rückstand wird aus Äthanol umkristallisiert. Man erhält 14,2 g 5-(3-tert. Butylamin-2-hydroxypropoxy)-2-pyridincarbonsäure, Dihydrochlorid vom Schmelzpunkt 161-167 °C.

Beispiel 4

5-(2-Hydroxy-3-isopropylaminopropoxy)-2-pyridincarbonsäurebenzylester, Hydrochlorid wird analog Beispiel 2 unter Verwendung von 1-Isopropylazetidinol hergestellt.

NMR-Spektrum ($\delta$; $D_2O$) : Dublett 1,3 (6 Protonen; J = 7 Hz), Multiplett 3,2-3,7 (3 Protonen), Multiplett 4,1-4,7 (3 Protonen), Singulett 5,4 (2 Protonen), Singulett 7,5 (5 Protonen), Dublett-Dublett 7,4 (1 Proton; J = 9 und 2,5 Hz), Dublett 8,0 (1 Proton; J = 9 Hz), Dublett 8,4 (1 Proton; J = 2,5 Hz)

IR-Spektrum (KBr): 3400 $cm^{-1}$ (NH); 1730 $cm^{-1}$ (C=O).

Beispiel 5

5-(2-Hydroxy-3-isopropylaminopropoxy)-2-pyridincarbonsäure, Dihydrochlorid wird analog Beispiel 3 aus dem Benzylester des Beispiels 4 hergestellt. NMR-Spektrum ($\delta$; $D_2O$) : Dublett 1,3 (6 Protonen; J = 7 Hz), Multiplett 3,2-3,6 (3 Protonen), Dublett-Dublett 8,2 (1 Proton; J = 9 und 2,5 Hz), Dublett 8,5 (1 Proton; J = 9 Hz), Dublett 8,6 (1 Proton; J = 2,5 Hz)

IR-Spektrum (KBr): 3500 $cm^{-1}$ (NH); 1720 $cm^{-1}$ (C=O).

Beispiel 6

Analog Beispiel 1d erhält man unter Verwendung von 5-Hydroxynicotinsäuremethylester 3-tert. Butyl-5-(5-methoxycarbonyl-3-pyridyloxymethyl)-2-phenyloxazolidinon (Kp. 130-150 °C/0,02 Torr), das mit konzentrierter Salzsäure 2 Stunden auf 100 °C erwärmt wird. Nach Erkalten wird mit Äther extrahiert und die wässrige Phase im Vakuum bis zur Trockne eingedampft. Aus Methylenchlorid/Äthanol erhält man 5-(3-tert. Butylamino-2-hydroxypropoxy)-3-pyridincarbonsäure, Dihydrochlorid vom Schmelzpunkt 246-251 °C.

Beispiel 7

a) 1,18 g 3-tert. Butyl-5-hydroxymethyl-2-phenyloxazolidin werden in 10 ml absolutem Dimethylformamid mit 150 mg einer 80 %igen Suspension von Natriumhydrid in Öl versetzt. Es wird 30 Minuten bei Raumtemperatur und 30 Minuten bei 100 °C gerührt und dann 855 mg 6-Chlornicotinsäuremethylester, gelöst in 3 ml Dimethylformamid, zugegeben. Man rührt 70 Stunden bei Raumtemperatur, gibt auf Wasser, äthert aus, wäscht die Ätherphase mehrfach mit Wasser, trocknet über Natriumsulfat

und dampft ein. Den Rückstand destilliert man im Kugelrohr, wobei ein Vorlauf bis 100 °C/0,02 Torr verworfen wird. Das gewünschte 3-tert. Butyl-5-(5-methoxycarbonyl-2-pyridyloxymethyl)-2-phenyloxazolidin destilliert bei 145-170 °C/0,02 Torr; Ausbeute 870 mg.

b) 200 mg 3-tert. Butyl-5-(5-methoxycarbonyl-2-pyridyloxymethyl)-2-phenyloxazolidin werden mit 3 ml konzentrierter Salzsäure 2 Stunden auf 100 °C erwärmt. Man äthert nach Erkalten und Verdünnen mit 3 ml Wasser aus, dampft die wässrige Phase im Vakuum ein und kristallisiert die gewünschte Säure aus Isopropanol um. Man erhält 90 mg 6-(3-tert. Butylamino-2-hydroxypropoxy)-3-pyridincarbonsäure vom Schmelzpunkt 215-220 °C.

Beispiel 8

200 mg des Oxazolidins aus Beispiel 7a werden mit 3 ml 1N Salzsäure 15 Minuten auf 100 °C erwärmt. Nach Erkalten extrahiert man mit Äther, sättigt die wässrige Phase mit Kaliumcarbonat und extrahiert mit Essigester. Nach Trocknen (Natriumsulfat) und Abdampfen des Lösungsmittels erhält man 97 mg 6-(3-tert. Butylamino-2-hydroxypropoxy)-3-pyridincarbonsäuremethylester, der nach Umkristallisation aus Methylenchlorid/Äther bei 131-134 °C schmilzt.

Analog Beispiel 7 werden des weiteren hergestellt:

3-(3-tert. Butylamino-2-hydroxypropoxy)-2-pyridincarbonsäure, Dihydrochlorid
Schmelzpunkt 158-177 °C (unter Zersetzung).

4-(3-tert. Butylamino-2-hydroxypropoxy)-2-pyridincarbonsäure, Dihydrochlorid
Stark hygroskopisch
NMR-Spektrum (δ; $D_2O$) : Singulett 1,5 (9 Protonen), Multiplett 3,2 - 4,7 (5 Protonen), Dublett-Dublett 7,7 [J = 7 und 3 Hz] (1 Proton), Dublett 8,05 [J = 3 Hz] (1 Proton), Dublett 8,75 [J = 7 Hz] (1 Proton).

6-(3-tert. Butalamino-2-hydroxypropoxy)-2-pyridincarbonsäure, Dihydrochlorid
Schmelzpunkt 120 - 125 °C.

4-(3-tert. Butylamino-2-hydroxypropoxy)-3-pyridincarbonsäure, Dihydrochlorid
Schmelzpunkt 197 - 202 °C.

4-(3-tert. Butylamino-2-hydroxypropoxy)-3-pyridincarbonsäure
Aus dem Dihydrochlorid durch Behandeln mit Anionaustauscher ($OH^-$-Form) in Methanol
Schmelzpunkt 252 - 254 °C.

3-(3-tert. Butylamino-2-hydroxypropoxy)-4-pyridincarbonsäure, Dihydrochlorid
Schmelzpunkt 183 - 185 °C.

2-(3-tert. Butylamino-2-hydroxypropoxy)-4-pyridincarbonsäure, Dihydrochlorid
Schmelzpunkt 114 - 115 °C.

Beispiel 9

Nach Beispiel 7α und 8 erhaltener 2-(3-tert. Butylamino-2-hydroxypropoxy)-3-pyridincarbonsäuremethylester wird mit methanolischem Bariumhydroxid 16 Stunden unter Rückfluß gekocht. Durch Einleiten von Kohlendioxid wird zunächst die Hauptmenge des Bariums entfernt. Nach Abfiltrieren wird das Filtrat stark eingeengt und eine Restmenge Barium durch Zugabe von methanolischer Schwefelsäure bis $p_H$ 5 gefällt. Nach Filtration wird durch Zugabe eines Anionaustauschers zum Filtrat sulfatfrei gemacht, der Austauscher abfiltriert und das Filtrat mit methanolischer Salzsäure angesäuert. Nach Abdampfen des Lösungsmittels erhält man aus Aceton 2-(3-tert. Butylamino-2-hydroxypropoxy)-3-pyridincarbonsäure, Dihydrochlorid vom Schmelzpunkt 80 -88 °C.

Beispiel 10

150 mg 5-(3-tert. Butylamino-2-hydroxypropoxy)-2-pyridincarbonsäurebenzylester (Beispiel 2) werden in 2 ml Pyridin gelöst und mit 0,036 ml Acetylchlorid unter Eiskühlung versetzt. Man rührt 15 Minuten in der Kälte, dann 20 Stunden bei Raumtemperatur, dampft im Vakuum ein und verteilt den Rückstand in Methylenchlorid/Sodalösung. Nach Abtrennen der organischen Phase, Trocknen (Natriumsulfat) und Abdampfen des Lösungsmittels verbleiben 100 mg 5-(3-tert. Butylamino-2-acetoxypropoxy)-2-pyridincarbonsäurebenzylester als ein Öl.
NMR-Spektrum (δ; Deuterotrichlormethan) Singulett 1,1 (9 Protonen), Singulett 2,1 (3 Protonen), Dublett 2,9

(2 Protonen) [J = 6 Hz], Dublett 4,3 (2 Protonen) [J = 5 Hz], Quintett 5,2 (1 Proton), Singulett 5,5 (2 Protonen), Multiplett 7,2 - 7,7 (6 Protonen), Dublett 8,1 (1 Proton) [J = 8 Hz], Dublett 8,5 (1 Proton) [J = 3 Hz].

## Patentansprüche

1. Substituierte Pyridine der allgemeinen Formel I

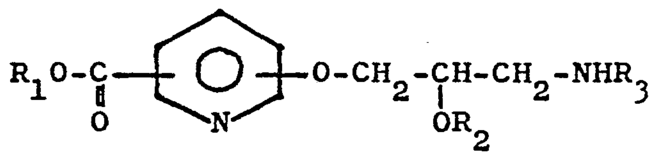 (I), worin

$R_1$ Wasserstoff, Alkyl mit bis zu 6 C-Atomen und Aralkyl mit 7-9 C-Atomen,

$R_2$ Wasserstoff und Alkanoyl mit 2 bis 7 C-Atomen und

$R_3$ Alkyl mit bis zu 6 C-Atomen bedeuten, sowie deren Additionssalze.

2. 5-(3-tert.-Butylamino-2-hydroxypropoxy)-2-pyridincarbonsäuremethylester und sein Dihydrochlorid

3. 5-(3-tert.-Butylamino-2-hydroxypropoxy)-2-pyridincarbonsäurebenzylester und sein Hydrochlorid

4. 5-(3-tert.-Butylamino-2-hydroxypropoxy)-2-pyridincarbonsäure und ihr Dihydrochlorid

5. 5-(2-Hydroxy-3-isopropylaminopropoxy)-2-pyridincarbonsäurebenzylester und sein Hydrochlorid

6. 5-(2-Hydroxy-3-isopropylaminopropoxy)-2-pyridincarbonsäure und ihr Dihydrochlorid

7. 5-(3-tert.-Butylamino-2-hydroxypropoxy)-3-pyridincarbonsäure und ihr Dihydrochlorid

8. 6-(3-tert.-Butylamino-2-hydroxypropoxy)-3-pyridincarbonsäure und ihr Dihydrochlorid

9. 6-(3-tert.-Butylamino-2-hydroxypropoxy)-3-pyridincarbonsäuremethylester und sein Dihydrochlorid

10. 3-(3-tert.-Butylamin-2-hydroxypropoxy)-2-pyridincarbonsäure und ihr Dihydrochlorid

11. 4-(3-tert.-Butylamino-2-hydroxypropoxy)-2-pyridincarbonsäure und ihr Dihydrochlorid

12. 6-(3-tert.-Butylamino-2-hydroxypropoxy)-2-pyridincarbonsäure und ihr Dihydrochlorid

13. 4-(3-tert.-Butylamino-2-hydroxypropoxy)-3-pyridincarbonsäure und ihr Dihydrochlorid

14. 5-(3-tert.-Butylamino-2-acetoxypropoxy)-2-pyridincarbonsäurebenzylester und sein Dihydrochlorid

15. 3-(3-tert.-Butylamino-2-hydroxypropoxy)-4-pyridincarbonsäure und ihr Dihydrochlorid

16. 2-(3-tert.-Butylamino-2-hydroxypropoxy)-4-pyridincarbonsäure und ihr Dihydrochlorid

17. 2-(3-tert.-Butylamino-2-hydroxypropoxy)-3-pyridincarbonsäure und ihr Dihydrochlorid

18. Verfahren zur Herstellung von Verbindungen der Formel I, dadurch gekennzeichnet, daß man in an sich bekannter Weise umsetzt

a) einen Hydroxyester der allgemeinen Formel II

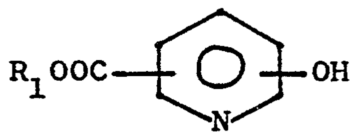

(II), worin

$R_1$ Alkyl mit bis zu 6 C-Atomen und Aralkyl mit 7-9 C-Atomen bedeutet,

mit einem Epoxid der Formel III

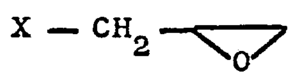

(III), worin

X ein Halogenatom, vorzugsweise Chlor, darstellt und anschließend mit einem primären Alkylamin mit bis zu 6 C-Atomen reagieren läßt

oder

b) einen Hydroxyester der allgemeinen Formel II mit einem Azetidinderivat der Formel IV

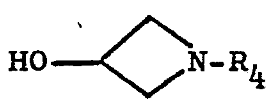

(IV), worin

$R_4$ einen Alkylrest mit bis zu 6 C-Atomen darstellt

oder

c) einen Hydroxyester der allgemeinen Formel II mit einem Oxazolidin der Formel V

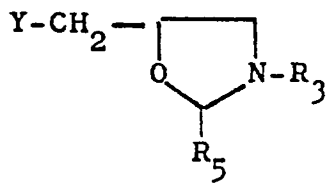 (V),

wobei $R_3$ die in Formel I angegebene Bedeutung hat und $R_5$ einen Alkyl- oder einen Arylrest mit bis zu 6 C-Atomen, vorzugsweise die Phenylgruppe darstellt und Y Tosyloxy oder Mesyloxy darstellt und anschließend mit einer verdünnten anorganischen Säure behandelt

oder

d) eine Verbindung der allgemeinen Formel VI

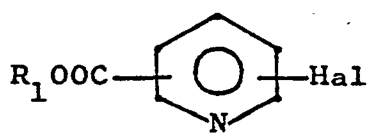 (VI), worin

$R_1$ die in Formel II angegebene Bedeutung hat, mit einem Epoxid der allgemeinen Formel VII

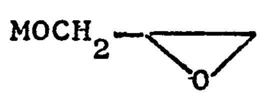 (VII),

wobei M ein Alkalimetall, vorzugsweise Natrium oder Kalium, darstellt und anschließend mit einem primären Alkylamin mit bis zu 6 C-Atomen reagieren läßt

oder

e) eine Verbindung der allgemeinen Formel VI mit einem Oxazolidin der Formel VIII

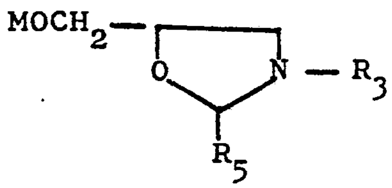

, worin

M, $R_3$ und $R_5$ die oben angegebene Bedeutung haben und anschließend mit einer verdünnten anorganischen Säure behandelt und gegebenenfalls Verbindungen der Formel I, in denen $R_1$ Alkyl oder Aralkyl bedeutet, sauer oder alkalisch hydrolysiert und in das entsprechende Säureadditionssalz oder Alkali- oder Erdalkalimetallsalz überführt.

und gegebenenfalls Verbindungen der Formel I, in denen $R_2$ Wasserstoff ist, mit einem reaktionsfähigen Säurederivat, vorzugsweise dem Säurechlorid oder Säureanhydrid, in Gegenwart eines Veresterungskatalysators acyliert.

19. Arzneimittel auf Basis von Verbindungen gemäß Anspruch 1 - 17.

0027990

Europäisches Patentamt

# EUROPÄISCHER RECHERCHENBERICHT

Nummer der Anmeldung
EP 80 10 6395

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | EP - A - 0 003 278 (MERCK)<br>* Ansprüche; Seite 4, Zeilen 5-13; Seite 14, Zeilen 15-30 *<br>-- | 1,18, 19 |
| | DE - A - 2 406 930 (CIBA-GEIGY)<br>* Ansprüche; Beispiele 28,35-37,62,65 *<br>-- | 1,18, 19 |
| D | DE - A - 2 556 110 (MERCK)<br>* Das ganze Dokument *<br>-- | 1,18, 19 |
| D | US - A - 4 053 605 (BALDWIN J.)<br>* Das ganze Dokument *<br>-- | 1,18, 19 |
| D | US - A - 4 092 419 (BALDWIN J.)<br>* Das ganze Dokument *<br>-- | 1,18, 19 |
| D | US - A - 4 091 104 (BALDWIN J.)<br>* Das ganze Dokument *<br>---- | 1,18, 19 |

KLASSIFIKATION DER ANMELDUNG (Int. Cl.³)

C 07 D 213/79
213/80
A 61 K 31/455
31/44//
C 07 D 213/85
413/12

RECHERCHIERTE SACHGEBIETE (Int. Cl.³)

C 07 D 213/79
213/80
A 61 K 31/44
31/455

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 22-01-1981 | NUYTS |